## Europäisches Patentamt
## European Patent Office
## Office européen des brevets

(19)

(11) Numéro de publication: **0 228 957**
**B1**

(12) **FASCICULE DE BREVET EUROPEEN**

(45) Date de publication du fascicule du brevet:
**05.04.89**

(21) Numéro de dépôt: **86402832.9**

(22) Date de dépôt: **16.12.86**

(51) Int. Cl.⁴: **C02F 1/26**, C07C 37/72

(54) Procédé d'extraction de composés organiques de leurs solutions ou suspensions aqueuses.

(30) Priorité: **18.12.85 FR 8518801**

(43) Date de publication de la demande:
**15.07.87 Bulletin 87/29**

(45) Mention de la délivrance du brevet:
**05.04.89 Bulletin 89/14**

(84) Etats contractants désignés:
**AT BE CH DE ES FR GB GR IT LI LU NL SE**

(56) Documents cités:
**FR-A- 710 948**
**FR-A- 2 383 126**
**US-A- 4 016 102**

(73) Titulaire: **ATOCHEM, 4 & 8, Cours Michelet La Défense 10, F-92800 Puteaux(FR)**

(72) Inventeur: **Benedetti, Charles, Pavillon 1 Cité La Reynarde Saint-Menet, F-13011 Marseille(FR)**
Inventeur: **Gluntz, Claude, Immeuble Santa Maria Les 3 Caravelles, F-13260 Cassis(FR)**
Inventeur: **Robert, Pascal, Bâtiment B 2 Résidence Central Park, F-13400 Aubagne(FR)**
Inventeur: **Stefanini, Michel, Bât. G Centre Sud 2 Avenue du Général de Gaulle, F-13380 Plan de Cuques(FR)**

(74) Mandataire: **Rochet, Michel et al, ATOCHEM Département Propriété Industrielle La Défense 10 4 & 8 Cours Michelet, F-92800 Puteaux(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'objet de la présente invention est un procédé d'extraction de composés organiques contenus dans de l'eau, sous forme de solutions et/ou suspensions.

Ce procédé s'applique, notamment, à la purification des eaux résiduaires contenant de tels composés et permet ainsi de résoudre un problème particulièrement difficile.

On sait, en effet, qu'il n'a jamais été facile de purifier, avant leur rejet, les eaux résiduaires provenant d'usines chimiques ou parachimiques et que cette difficulté augmente avec la sévérité des normes imposées par les différentes législations destinées à protéger l'environnement, notamment celles relatives à la demande chimique en oxygène (DCO).

De nombreuses méthodes ont déjà été proposées pour purifier de telles eaux; citons, entre autres, celles fondées sur l'oxydation, le passage sur des substances adsorbantes solides (brevet français n° 8 415 174), la centrifugation (brevet français n° 7 419 446).

On a également fait appel à l'extraction liquide-liquide; mais, les procédés, fondés sur ce principe, requièrent l'emploi de composés chimiques souvent coûteux, parfois toxiques et semblent ne pouvoir s'appliquer qu'à des cas particuliers: c'est ainsi que les solutions d'amines ont été proposées pour extraire les acides humiques (Vom Wasser – 1973 – 41–27–44), le 2-éthyl hexanol pour épurer les eaux résiduaires provenant de la fabrication des dialkylphtalates (Informations Chimie – N° 136 – Octobre 1974 – pages 193 à 197).

De plus, la solubilité de ces composés dans les eaux résiduaires est parfois loin d'être négligeable rendant, de ce fait, le rejet des eaux purifiées plus que problématique, surtout si le composé utilisé n'est pas biodégradable.

Le procédé qui fait l'objet de la présente invention fait appel à une technique d'extraction liquide-liquide qui ne présente pas les inconvénients signalés ci-dessus.

Ce procédé consiste à, tout d'abord, traiter ladite eau par un acide organique carboxylique, liquide et immiscible à la température de traitement, ce qui conduit à la formation de deux phases séparées, l'une organique et l'autre aqueuse et, ensuite, à séparer ces deux phases.

Les composés, contenus initialement dans l'eau, se répartissent, selon un certain coefficient de partage, entre ces deux phases.

Si nécessaire, on peut procéder à plusieurs extractions par l'acide.

Les composés, dissous dans la phase organique, peuvent ensuite en être séparés et, par suite, récupérés, par toute méthode convenable – par distillation, par exemple, s'ils sont volatils.

Comme dit ci-dessus, l'acide carboxylique doit être immiscible avec l'eau; en d'autres termes, il doit être insoluble ou peu soluble dans l'eau.

On utilisera, de préférence, un acide dont la solubilité est au maximum égale à 5 grammes par litre à 20°C.

Cet acide peut être aliphatique, linéaire ou ramifié, saturé ou insaturé, cyclique, hétérocyclique ...; il peut contenir des hétéroatomes tels que soufre, phosphore, azote.

On peut, bien entendu, utiliser également dans l'invention un mélange d'acides.

Conviennent particulièrement bien les acides: n-heptanoïque, éthyl-2-hexanoïque, isopropylacétique, n-nonanoïque appelé pélargonique ou leurs mélanges.

L'acide n-heptanoïque, facilement biodégradable, est particulièrement intéressant.

Parmi les composés contenus dans les eaux à traiter et susceptibles d'être extraits quantitativement par le ou les acides, citons: les amines, les amino-acides, les lactames et, ce qui est plus surprenant encore, les phénols.

Lorsque le composé à extraire est peu soluble dans l'eau et qu'il y est présent en quantité excédant cette solubilité, on a, à la fois, une solution et une suspension.

Pour réaliser cette extraction, on utilise les méthodes habituelles d'extraction liquide-liquide; on peut, par exemple, après avoir mis l'acide dans la solution ou suspension aqueuse, contenue dans un récipient, soumettre le tout à une forte agitation de manière à disperser l'acide sous forme de gouttelettes puis laisser décanter; on peut, également, pratiquer une extraction à contre-courant dans une colonne verticale.

L'extraction peut se faire à n'importe quelle température; des essais préalables de laboratoire permettront de déterminer quelle est la température optimale pour chaque cas particulier.

Dans les exemples qui vont suivre, exemples donnés à titre d'illustration et sans le moindre caractère limitatif, les solutions ou suspensions aqueuses ont été préparées en ajoutant à un litre d'eau, sous agitation modérée, de 1 à 2 pour cent (soit, de 10 à 20 grammes) du composé organique étudié, cette eau étant contenue dans un réacteur muni d'une double enveloppe, parcourue par un fluide caloporteur, permettant de maintenir une température donnée.

Puis, l'acide carboxylique est introduit à raison de 2 ou 4,6 parties en poids pour cent d'eau – après 10 minutes d'agitation suivie de décantation, les deux phases, c'est-à-dire, la phase organique surnageante et la phase aqueuse, sont séparées, pesées et la quantité de composé organique non extrait déterminée par analyse dans la phase aqueuse – soit, par évaporation (détermination de l'extrait sec) soit par dosage potentiométrique à l'acide chlorhydrique dans le cas de composés aminés.

Si on appelle:

– Q, la masse du composé organique à extraire mis en œuvre,
– X, sa teneur en pour cent, en poids, dans la phase aqueuse après extraction,
– Y, sa teneur en pour cent, en poids, dans la phase organique après extraction,
– M, la masse de la phase aqueuse après extraction,
– m, la masse de la phase organique après extraction,
– Kd, le coefficient de partage du composé organique entre la phase organique et la phase aqueuse, après extraction,
– Rd, le rendement d'extraction du composé, en pour cent.

On aura:

$$Q = Y \cdot \frac{m}{100} + X \cdot \frac{M}{100}$$

soit:

$$Y = \left(Q - X \cdot \frac{M}{100}\right) \cdot \frac{100}{m}$$

$$Kd = \frac{Y}{X}$$

$$Rd \text{ (en pour cent)} = \frac{Y \cdot m}{Q}$$

Le tableau n° I ci-après rassemble les résultats d'essais d'extraction réalisés à la température de 60°C avec de l'acide n-heptanoïque sur divers composés.

### Tableau I

| Composition de la solution ou suspension aqueuse initiale | | | Résultats après extraction | | | |
|---|---|---|---|---|---|---|
| Nature du composé à extraire | Teneur en composé en % | Teneur en acide en % | X en pour cent | Y en pour cent | Coefficient de partage Kd | Rendement Rd en pour cent |
| Phénol | 1 | 2 | 0,5 | 20 | 33 | 50 |
| 2-3 Dichlorophénol | 2 | 4,6 | 0,118 | 32 | 299 | 94,5 |
| Ortho-chlorophénol | 2 | 4,6 | 0,0019 | 99,91 | 21910 | 99,9 |
| Di-éthyl-amine | 2 | 4,6 | 1,7 | 22,4 | 13 | 12,5 |
| Tri-éthyl-amine | 1 | 2 | 0,85 | 29,4 | 35 | 15 |
| Heptylamine | 1 | 2 | 0,35 | 27,5 | 79 | 65 |
| N-N-diméthyl-benzyl-amine | 2 | 4,6 | 0,16 | 40,8 | 252 | 91,9 |
| Hexylamine | 2 | 4,6 | 0,135 | 31,1 | 230 | 93,3 |
| Di-n-hexylamine | 2 | 4,6 | 0,003 | 91,5 | 10500 | 99,9 |
| Acide 11-aminoundécanoïque | 2 | 4,6 | 0,48 | 25,36 | 52,83 | 77,35 |
| Acide 12-aminododécanoïque | 1 | 2 | 0,3 | 25,9 | 86 | 70 |
| Dodécanolactame | 2 | 4,6 | 0,1 | 30,22 | 302 | 95,2 |

Le tableau n° II ci-après rassemble les résultats d'extraction effectués.
– à deux températures différentes: 25 et 60°C,
– sur des compositions aqueuses dont la composition intiale à 25°C est de 20 grammes d'acide 11-aminoundécanoïque pour 1 litre d'eau,
– au moyen de divers acides dont l'acide EMERY 12–10 de la Société EMERY (mélange d'acides hexanoïque et octanoïque) à raison de 5 pour cent en volume par rapport à l'eau mise en œuvre soit 50 cc, tous ces acides ayant une solubilité dans l'eau inférieure à 5 grammes par litre à 20°C.

EP 0 228 957 B1

| Tableau II | | | | |
|---|---|---|---|---|
| Nature de l'acide | 25°C | | 60°C | |
| | Kd | Rd | Kd | Rd |
| n-heptanoïque | 76,8 | 81,7 | 52,8 | 77,3 |
| n-octanoïque | 187,7 | 92,9 | 124,8 | 89,2 |
| 2 éthyl-hexanoïque | 288 | 95,1 | 91,3 | 84,7 |
| n-nonanoïque | 156,1 | 91,2 | 46,7 | 73,2 |
| Emery 12-10 | 87,5 | 84,2 | 56,3 | 77,4 |

Le tableau n° III ci-après permet de comparer le pouvoir extracteur des acides n-heptanoïque et n-octanoïque, à 60°C, sur deux composés différents: le dodécanolactame et le 2,3-dichlorophénol.

| Tableau III | | | | |
|---|---|---|---|---|
| Nature de l'acide | Nature du composé extrait | | | |
| | Dodécanolactame | | 2-3-di-chloro-phénol | |
| | Kd | Rd | Kd | Rd |
| n-heptanoïque | 302 | 95,2 | 299 | 94,5 |
| n-octanoïque | 344 | 95,7 | 22200 | 99,9 |

## Revendications

1. Procédé d'extraction de composés organiques de leurs solutions et/ou suspensions aqueuses, caractérisé en ce qu'il consiste à traiter lesdites solutions et/ou suspensions par un acide organique carboxylique, liquide à la température de traitement et immiscible avec l'eau à 20°C, et à isoler la phase organique, contenant le composé extrait, de la phase aqueuse.

2. Procédé selon la revendication 1, caractérisé en ce que l'acide organique possède une solubilité au maximum égale à 5 grammes par litre d'eau à 20°C.

3. Procédé selon la revendication 2, caractérisé en ce que l'acide est choisi dans le groupe constitué par les acides n-heptanoïque, n-octanoïque, 2-éthyl-hexanoïque, isopropylacétique et n-nonanoïque.

4. Procédé selon l'une quelconque des revendications 1 à 3, caractérisé en ce que le composé à extraire est une amine ou un amino-acide.

5. Procédé selon la revendication 4, caractérisé en ce que le composé à extraire est choisi dans le groupe constitué par l'heptylamine, la N,N-diméthylbenzylamine, l'hexylamine, la di-n-hexylamine, l'acide 11-aminoundécanoïque et l'acide 12-aminododécanoïque.

6. Procédé selon l'une quelconque des revendications 1 à 3, caractérisé en ce que le composé à extraire est le dodécanolactame.

7. Procédé selon l'une quelconque des revendications 1 à 3, caractérisé en ce que le composé à extraire est un phénol.

8. Procédé selon la revendication 7, caractérisé en ce que le composé à extraire est choisi dans le groupe constitué par le 2,3-dichlorophénol et l'ortho-chlorophénol.

9. Application du procédé selon l'une quelconque des revendications 1 à 8 à l'épuration d'eaux résiduaires industrielles.

## Patentansprüche

1. Verfahren zur Extraktion organischer Verbindungen aus deren wässrigen Lösungen und/oder Suspensionen, dadurch gekennzeichnet, daß es darin besteht, diese Lösungen und/oder Suspensionen mit einer organischen Carbonsäure zu behandeln, die bei der Behandlungstemperatur flüssig ist und mit Wasser bei 20°C nicht mischbar ist, und die organische Phase, die die extrahierte Verbindung enthält, von der wässrigen Phase zu isolieren.

2. Verfahren nach Anspruch 1, dadurch gekennzeichnet, daß die organische Säure eine Löslichkeit von maximal 5 g/l Wasser bei 20°C aufweist.

3. Verfahren nach Anspruch 2, dadurch gekennzeichnet, daß die Säure ausgewählt wird aus der Gruppe bestehend aus den N-Heptansäuren, N-Octansäuren, 2-Ethylhexansäuren, Isopropylessigsäuren und N-Nonansäuren.

4. Verfahren nach einem der Ansprüche 1 bis 3, dadurch gekennzeichnet, daß die zu extrahierende Verbindung ein Amin oder eine Aminosäure ist.

4

5. Verfahren nach Anspruch 4, dadurch gekennzeichnet, daß die zu extrahierende Verbindung ausgewählt wird aus der Gruppe bestehend aus Heptylamin, N,N-Dimethylbenzylamin, Hexylamin, Di-n-hexylamin, 11-Aminoundecansäure und 12-Aminododecansäure.

6. Verfahren nach einem der Ansprüche 1 bis 3, dadurch gekennzeichnet, daß die zu extrahierende Verbindung Dodecanolactam ist.

7. Verfahren nach einem der Ansprüche 1 bis 3, dadurch gekennzeichnet, daß die zu extrahierende Verbindung ein Phenol ist.

8. Verfahren nach Anspruch 7, dadurch gekennzeichnet, daß die zu extrahierende Verbindung ausgewählt wird aus der Gruppe bestehend aus 2,3-Dichlorphenol und ortho-Chlorphenol.

9. Verwendung eines Verfahrens nach einem der Ansprüche 1 bis 8 zur Reinigung von industriellen Abwässern.

**Claims**

1. Process for the extraction of organic compounds from their aqueous solutions and/or suspensions, characterized in that it consists of treating the said solutions and/or suspensions with a carboxylic organic acid which is liquid at the treatment temperature and immiscible with water at 20°C, and isolating the organic phase, containing the extracted compound, from the aqueous phase.

2. Process according to claim 1, characterized in that the organic acid has a solubility at most equal to 5 grams per litre of water at 20°C.

3. Process according to claim 2, characterized in that the acid is chosen from the group consisting of n-heptanoic acid, n-octanoic acid, 2-ethyl-hexanoic acid, isopropylacetic acid and n-nonanoic acid.

4. Process according to any one of claims 1 to 3, characterized in that the compound to be extracted is an amine or an aminoacid.

5. Process according to claim 4, characterized in that the compound to be extracted is chosen from the group consisting of heptylamine, N,N-dimethylbenzylamine, hexylamine, di-n-hexylamine, 11-aminoundecanoic acid and 12-aminododecanoic acid.

6. Process according to any one of claims 1 to 3, characterized in that the compound to be extracted is dodecanolactam.

7. Process according to any one of claims 1 to 3, characterized in that the compound to be extracted is a phenol.

8. Process according to claim 7, characterized in that the compound to be extracted is chosen from the group consisting of 2,3-dichlorophenol and orthochlorophenol.

9. Application of the process according to any one of claims 1 to 8 to the purification of industrial waste waters.